# EUROPEAN PATENT SPECIFICATION

(11) **EP 4 489 971 B1**
(45) Date of publication and mention of the grant of the patent: **17.12.2025**
(21) Application number: 23707717.7
(22) Date of filing: 27.02.2023
(51) Int. Cl.: B41J 11/04, A61B 5/344, B41J 2/32, B41J 25/308, B41J 25/312, B41J 29/02

(54) **PRINTING MECHANISM FOR MEDICAL MONITORING DEVICE AND MEDICAL MONITORING DEVICE**
DRUCKMECHANISMUS FÜR MEDIZINISCHE ÜBERWACHUNGSVORRICHTUNG UND MEDIZINISCHE ÜBERWACHUNGSVORRICHTUNG
MÉCANISME D'IMPRESSION POUR DISPOSITIF DE SURVEILLANCE MÉDICALE ET DISPOSITIF DE SURVEILLANCE MÉDICALE

(30) Priority: 07.03.2022 CN 202210217248; 07.03.2022 CN 202220480206 U
(43) Date of publication of application: 15.01.2025
(73) Proprietor: Koninklijke Philips N.V., 5656 AG Eindhoven (NL)
(72) Inventor: YANG, Lei, 5656 AG Eindhoven (NL); TANG, Liang Yong, 5656 AG Eindhoven (NL)
(74) Representative: Philips Intellectual Property & Standards
(86) International application number: PCT/EP2023/054777
(87) International publication number: WO 2023/169850

(56) References cited:
- US-A1- 2002 024 578
- US-A1- 2007 291 100

## Description

### FIELD OF THE INVENTION

The present invention relates to a printing mechanism for a medical monitoring device and a medical monitoring device comprising such a printing mechanism.

### BACKGROUND OF THE INVENTION

A medical monitoring device such as a fetal monitor, an electrocardiograph, a defibrillator or an ultrasound diagnostic device generally comprises a main unit and a printing mechanism disposed in the main unit. When the printing mechanism of the medical monitoring device for example a fetal monitor operates, a rubber platen driven by a stepper motor rotates at a preset speed to drag a print paper past a thermal printing head to record a curve characterizing fetal vital characteristics on thermal print paper. In the existing printing mechanisms, the stepper motor and the rubber platen are mounted on different components to form separable structures. Usually, a gear is mounted on an end of a rotating shaft of the stepper motor and an end of a shaft of the rubber platen, and the stepper motor causes the rubber platen to rotate through engagement between the gears, which in turn drags the print paper pressed between the rubber platen and the printing head. When the printing mechanism operates, the rubber platen and the printing head need to be pressed together at appropriate pressure to ensure that the print paper passing between them fits accurately with the printing head. Usually, the rubber platen as a movable part follows a print paper drawer to perform opening and closing action, a printing head mounting frame (a printing head mounting portion) is fixed to a housing of the medical monitoring device, and the printing head is held down by a set of springs to allow for a small amount of movement (a floating design). When the printing head contacts the rubber platen, the springs provide the compression force to cause the printing head to press against the rubber platen. In the existing structure, the elasticity of the springs changes as the printing mechanism is used, resulting in a change in the compression force applied between the rubber platen and the printing head and thus poor printing quality on the print paper. In addition, the set of springs pressing the printing head down also occupies a large space in the medical monitoring device.

Therefore, there is a need to improve the existing printing mechanism for the medical monitoring device. Documents US 2007/291100 A1 and US 2002/024578 A1 represent relevant prior art for the present invention.

### SUMMARY OF THE INVENTION

It is an object of the present invention to provide a printing mechanism for a medical monitoring device which can overcome at least one of the above-mentioned disadvantages in the prior art.

According to the present invention, there is provided a printing mechanism for a medical monitoring device comprising:
a support plate;
a L-shaped support arm rotatably disposed on the support plate at one end, the support arm provided with a printing head mounting portion at the other end, the printing head mounting portion provided with a printing head thereon; and
a movable frame assembly movably disposed on the support plate and between the support plate and the support arm, the movable frame assembly comprising a frame, a rubber platen mounted at a front end of the frame adjacent to the outside of the medical monitoring device to be in contact with the printing head under pressure, a print paper drawer mounted on the frame, and a driving mechanism mounted on the frame to drive the rubber platen to rotate;
wherein the frame is formed with a guide slot having a curved contour therein, and a guide pin extending from both sides of the printing head mounting portion is movably received in the respective guide slot to limit a relative position and a compression force between the printing head and the rubber platen.

Preferably, the guide slot may comprise a first guide slot and a second guide slot formed oppositely in the frame, the first guide slot and the second guide slot have a curved contour that rises gradually from a front end to a rear end thereof.

Preferably, the first guide slot and the second guide slot may each have a notch formed at a front end thereof, and the printing head presses against the rubber platen when the guide pin is moved into the notch.

Preferably, the length of the first guide slot and the second guide slot may be selected to be equal to the maximum travel of the movable frame assembly when the movable frame assembly is pulled out of a housing of the medical monitoring device, and the height (H) of the first guide slot and the second guide slot may be selected to determine a maximum rotation angle of the printing head and the support arm.

Preferably, the frame may comprise a first mounting plate and a second mounting plate disposed opposite to each other, and a first connecting plate and a second connecting plate connecting fixedly the first mounting plate and the second mounting plate together, the first guide slot is formed in the first mounting plate and the second guide slot is formed in the second mounting plate.

Preferably, the guide pin is provided with a rolling bearing thereon, and the guide pin may be movably received in the respective guide slot by the rolling bearing.

Preferably, a reset spring is further provided, and the reset spring tends to cause the printing head to rotate and press against the rubber platen.

Preferably, the driving mechanism may comprise a first pulley fixedly mounted at one end of the rubber platen, an electric motor mounted on the frame, a second pulley mounted on a rotating shaft of the electric motor, and a timing belt for connecting the first pulley and the second pulley so as to drive the rubber platen to rotate when the electric motor operates.

Preferably, the electric motor is disposed at a rear end of the frame adjacent to the inside of the medical monitoring device.

Preferably, the rubber platen may be rotatably supported by bearings at a front end of the first mounting plate and the second mounting plate adjacent to the outside of the medical monitoring device, the electric motor is spaced apart from the rubber platen along a longitudinal direction of the first mounting plate and is mounted at a rear end of the first mounting plate adjacent to the inside of the medical monitoring device.

Preferably, the first pulley and the second pulley may be disposed adjacent to the first mounting plate.

Preferably, the first mounting plate, the second mounting plate, the first connecting plate and the second connecting plate are metal or alloy members which are joined together to form a rigid frame .

Preferably, the medical monitoring device may be a fetal monitor.

According to another aspect of the present invention, there is provided a medical monitoring device, wherein the medical monitoring device comprises a printing mechanism for a medical monitoring device as above stated.

According to the printing mechanism for the medical monitoring device of the present invention, the curved contour of the guide slots ensures that the printing head and the rubber platen accurately cooperate with each other, and the rubber platen is subjected to the appropriate pressure and the appropriate deformation so as to achieve excellent printing quality. On the other hand, the rotation amplitude of the printing head and the support arm is limited, thereby not affecting the layout of the medical monitoring device, and not occupying too much space of the medical monitoring device.

### BRIEF DESCRIPTION OF THE DRAWINGS

FIG. 1 schematically shows in a perspective view a printing mechanism for a medical monitoring device according to the present invention;
FIG. 2 shows schematically in another perspective view the printing mechanism for the medical monitoring device according to the present invention, wherein an electric motor has been removed from a frame;
FIG. 3 shows schematically in another perspective view the printing mechanism for the medical monitoring device according to the present invention, wherein an electric motor and a support arm have been removed from a frame;
FIG. 4 shows schematically in a perspective view a movable frame assembly of a printing mechanism for a medical monitoring device according to the present invention, wherein an electric motor and a rubber platen are mounted on the frame;
FIG. 5 schematically shows in a perspective view a movable frame assembly of a printing mechanism for a medical monitoring device according to the present invention, wherein a rubber platen and a print paper drawer are mounted on the frame;
FIG. 6 shows schematically in a perspective view a support arm and a printing head mounting portion of a printing mechanism for a medical monitoring device according to the present invention;
FIG. 7 is a side view of the structure shown in FIG. 6; and
FIG. 8 is a side view of the structure shown in FIG. 2 with a front decorative plate removed.

### DETAILED DESCRIPTION OF THE EXEMPLARY EMBODIMENTS

Preferred embodiments of the present invention are described in detail hereinafter in connection with the drawings. It should be understood by those skilled in the art that the drawings are intended to illustrate the invention only and are not meant to form any limit to the present invention.

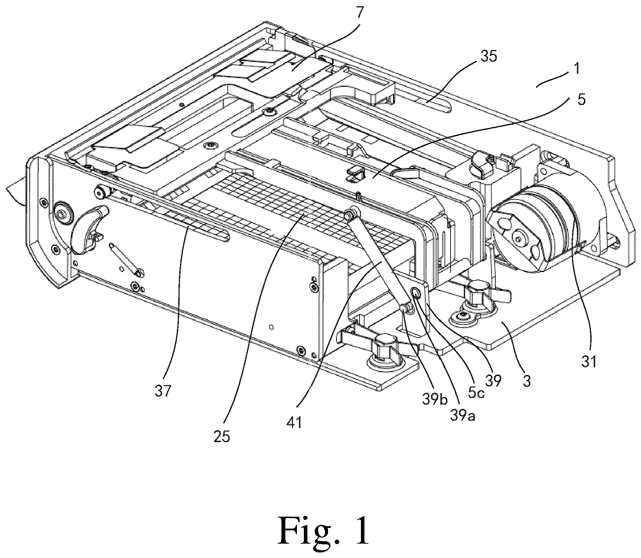

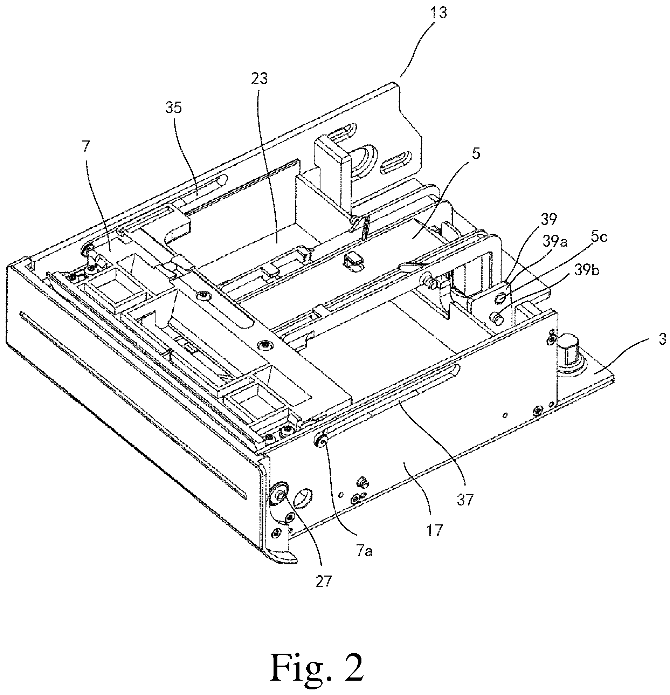

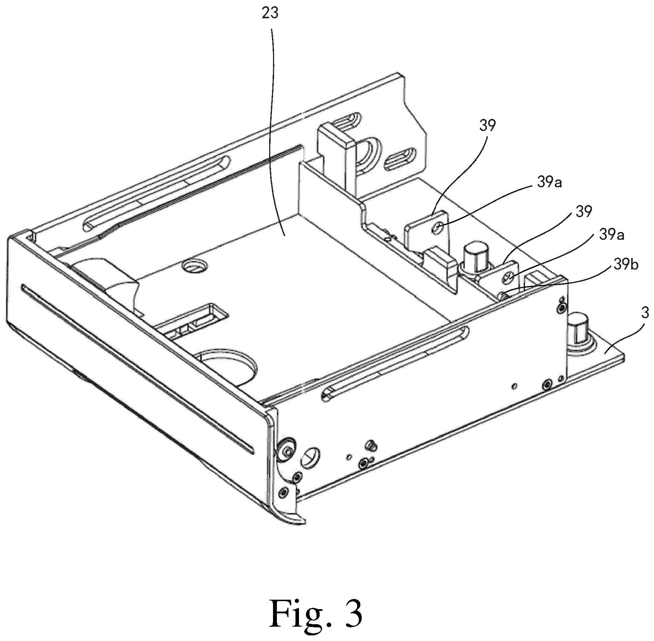

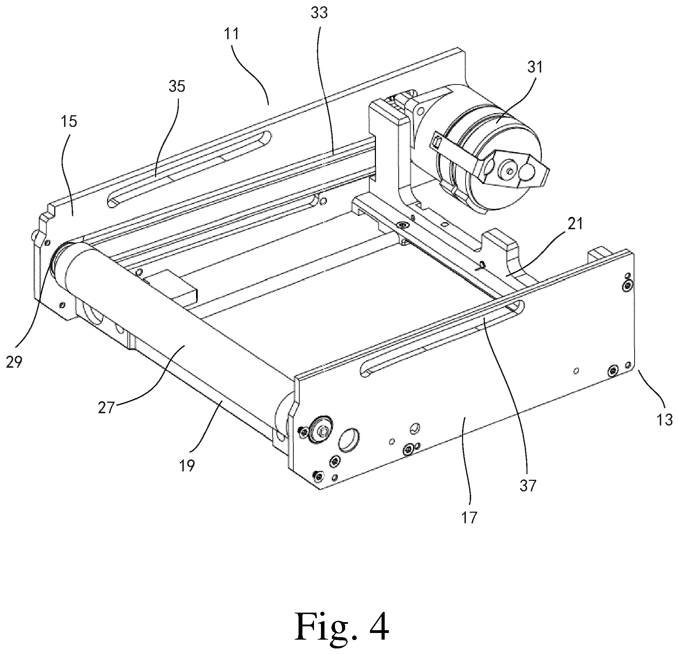

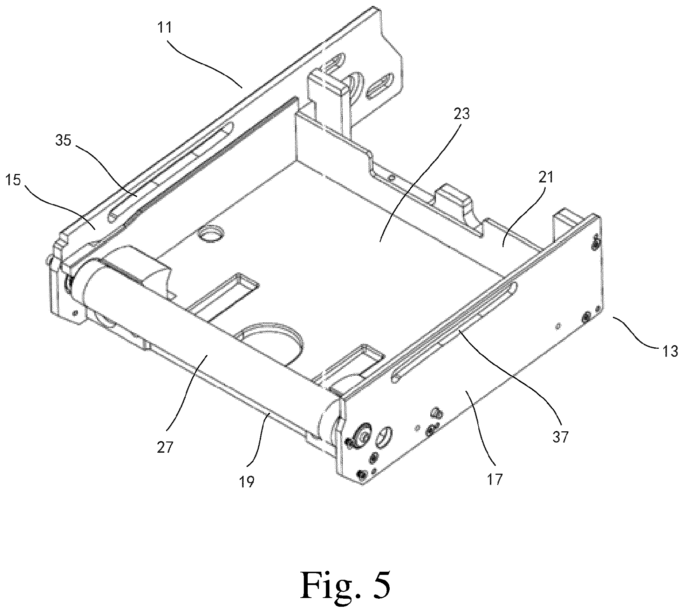

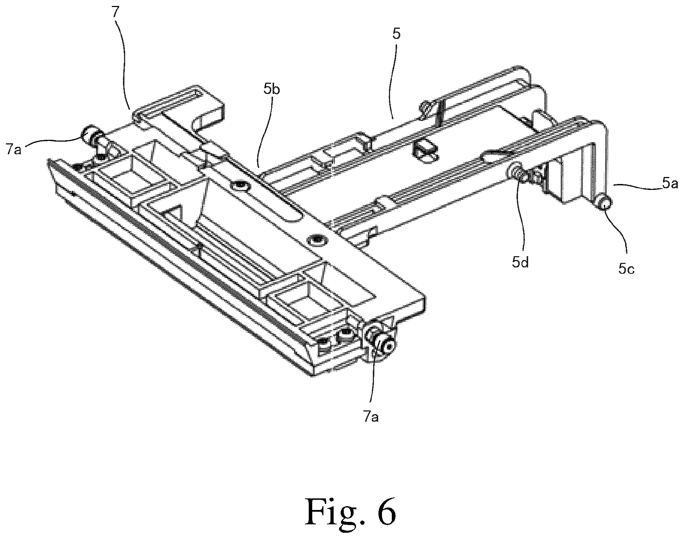

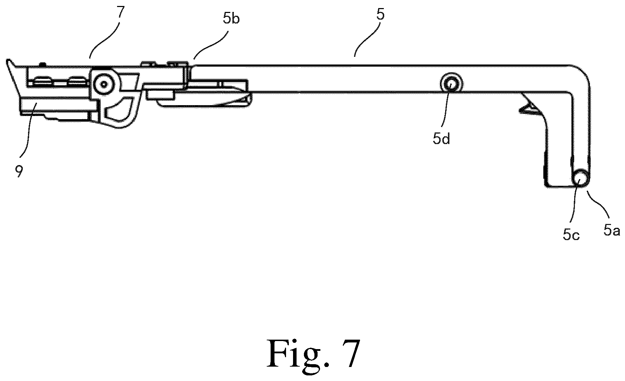

FIG. 1 schematically shows in a perspective view a printing mechanism for a medical monitoring device according to the present invention; FIG. 2 shows schematically in another perspective view the printing mechanism for the medical monitoring device according to the present invention, wherein an electric motor has been removed from a frame; FIG. 3 shows schematically in another perspective view the printing mechanism for the medical monitoring device according to the present invention, wherein an electric motor and a support arm have been removed from a frame; FIG. 4 shows schematically in a perspective view a movable frame assembly of a printing mechanism for a medical monitoring device according to the present invention, wherein an electric motor and a rubber platen are mounted on the frame; FIG. 5 schematically shows in a perspective view a movable frame assembly of a printing mechanism for a medical monitoring device according to the present invention, wherein a rubber platen and a print paper drawer are mounted on the frame; FIG. 6 shows schematically in a perspective view a support arm and a printing head mounting portion of a printing mechanism for a medical monitoring device according to the present invention; and FIG. 7 is a side view of the structure shown in FIG. 6. As shown in FIGS. 1-7, a printing mechanism 1 for a medical monitoring device according to the present invention generally comprises a support plate (or a base plate) 3 which is typically mounted on a housing of the medical monitoring device (not shown) such as a fetal monitor. The printing mechanism 1 for the medical monitoring device according to the present invention further comprises a support arm 5. In a preferred embodiment, the support arm 5 is substantially L-shaped, one end portion 5a of the support arm 5 is rotatably mounted on the support plate 3, and the other end 5b of the support arm 5 is provided with a printing head mounting portion 7 on which a printing head 9 for example a thermal printing head is mounted. In FIG. 1, the printing head is invisible because it is mounted on the underside of the printing head mounting portion 7. The printing head is electrically communicated with a main unit of the medical monitoring device by wire or wirelessly, and thus receives signals from the main unit to perform the printing operations. The printing mechanism 1 for the medical monitoring device according to the present invention also comprises a movable frame assembly 11, which is movably disposed on the support plate 3 and in a space between the support plate 3 and the support arm 5.

As shown in FIGS. 4 and 5, the movable frame assembly 11 comprises a frame 13. The frame 13 comprises a first mounting plate 15 and a second mounting plate 17 disposed opposite to each other, and a first connecting plate 19 and a second connecting plate 21 that connect fixedly the first mounting plate 15 and the second mounting plate 17 together. In the preferred embodiment, the first mounting plate 15, the second mounting plate 17, the first connecting plate 19, and the second connecting plate 21 are metal members such as aluminum or other lightweight metal or alloy members, which are formed separately and then connected together by fasteners such as screws so that the frame 13 is formed as a highly rigid metal frame. However, it should be understood that the first mounting plate 15, the second mounting plate 17, the first connecting plate 19, and the second connecting plate 21 can also be cast in one piece from metal. Of course, the first mounting plate 15, the second mounting plate 17, the first connecting plate 19 and the second connecting plate 21 can also be molded in one piece from plastic. The movable frame assembly 11 also comprises a print paper drawer 23 mounted between the first mounting plate 15 and the second mounting plate 17 to hold the print papers 25. In FIG. 4, the print paper drawer mounted on the frame 13 is not shown.

The movable frame assembly 11 also comprises a rubber platen 27 whose ends are rotatably supported on the first mounting plate 15 and the second mounting plate 17. The rubber platen 27 is rotatably supported at the front end of the first mounting plate 15 and the second mounting plate 17 adjacent to the outside of the medical monitoring device by bearings, and a first pulley 29 is fixedly mounted at one end of the rubber platen 27. The movable frame assembly 11 also comprises an electric motor 31, which is spaced apart from the rubber platen 27 along a longitudinal direction of the first mounting plate 15 and mounted on the first mounting plate 15, and a second pulley (invisible in the drawings) is mounted on a rotating shaft of the electric motor 31. A timing belt 33 connects the first pulley 29 and the second pulley so that the electric motor drives the rubber platen 27 to rotate by means of the second pulley, the timing belt 33, and the first pulley 29 when the electric motor operates. Thus, according to the present invention, the rubber platen 27, the electric motor 31, and the timing belt 33 connecting the rubber platen 27 and the electric motor 31 are all mounted on the frame 13. In this way, even though the movable frame assembly 11 is pulled out of the housing of the medical monitoring device to pick up and add the print paper and then is inserted into the housing of the medical monitoring device 13, the rubber platen 27, the electric motor 31, and the timing belt 33 move together with the frame but their relative positions remain unchanged, thus ensuring that they are positioned accurately, which in turn allows the printing mechanism to operate smoothly without generating noise or generating significantly reduced noise.

Preferably, the electric motor 31 is disposed at a rear end of the first mounting plate 15 adjacent to the inside of the medical monitoring device, which allows the movable frame assembly 11 to be formed with a substantially uniform thickness and thus have a relatively flat and regular shape. As a result, the printing mechanism may have a relatively compact and regular layout. More preferably, the first pulley 29, the second pulley, and the electric motor 31 are all provided adjacent to the first mounting plate 15, thus making the overall structure more compact and occupying as little space as possible. The first mounting plate 15 and the second mounting plate 17 are sized such that the rubber platen 27, the first pulley on the rubber platen 27, the timing belt 33, the electric motor 31, and the second pulley on the electric motor 31 are all located inside of at least one of the first mounting plate 15 and the second mounting plate 17, ensuring that no moving component protrudes out of the frame 13 or is exposed on the outside of the frame 13. Although the first mounting plate 15 and the second mounting plate 17 may have the same length, in the preferred embodiment the length of the second mounting plate 17 may be shorter than that of the first mounting plate 15 because the second mounting plate 17 does not have to support the electric motor 31, which reduces the amount of material used and lowers the weight of the frame 13. A first guide slot 35 and a second guide slot 37 extending along the longitudinal direction and having a curved contour are formed in the first mounting plate 15 and the second mounting plate 17 respectively.

As shown in FIGS. 1 and 3, there are also a pair of mounting seats 39 disposed oppositely on the support plate 3. Each of the mounting seats 39 is formed with a mounting hole 39a and a first projection 39b formed on the outside. As shown in FIGS. 6 and 7, the generally L-shaped support arm 5 has a protruding assembly shaft 5c formed on both sides of the end portion 5a, and a second projection 5d is formed on both sides of a main portion of the generally L-shaped support arm 5 (i.e., the portion located above the print paper drawer). A guide pin 7a extends outward from both sides of the print head mounting portion 7.

During assembly, the assembly shafts 5c formed at the end portion 5a of the support arm 5 are rotatably received in the mounting holes 39a in the mounting seats 39 respectively, and the guide pins 7a extending outward from both sides of the print head mounting portion 7 are movably received in the first guide slot 35 in the first mounting plate 15 and the second guide slot 37 in the second mounting plate 17 respectively. Preferably, each guide pin 7a is equipped with a rolling bearing, and the guide pins 7a are received in the first guide slot 35 in the first mounting plate 15 and the second guide slot 37 in the second mounting plate 17 by the rolling bearings. Further, one end of a reset spring 41 is attached to the first projection 39b on the mount seat 39, and the other end of the reset spring 41 is attached to the second projection 5d on the support arm 5.

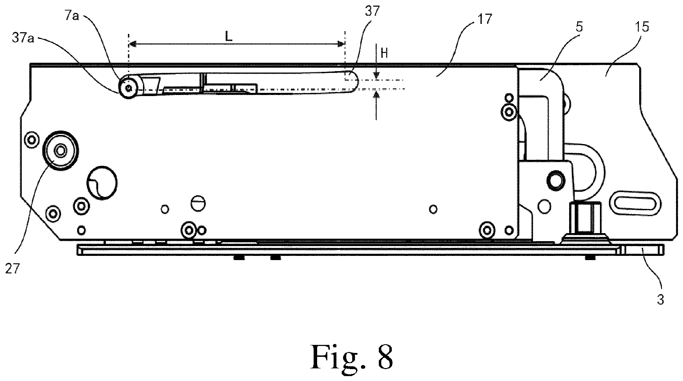

FIG. 8 is a side view of the structure shown in FIG. 2 with a front decorative plate removed. As shown in FIG. 8, the first guide slot 35 and the second guide slot 37 have a curved contour that rises gradually from the front end (the end adjacent to the rubber platen 27) to the rear end so that the printing head mounting portion 7 provided with the printing head 9 is lifted off the rubber platen 27 against the action of the reset spring 41 as the movable frame assembly 11 is pulled outwardly (to the left in FIG. 7) from the housing of the medical monitoring device. A notch is formed at the front end of the first guide slot 35 and the second guide slot 37 (only the notch 37a formed at the front end of the second guide slot 37 is shown in FIG. 8). When the movable frame assembly 11 is pushed into the housing of the medical monitoring device (the movable frame assembly 11 is pushed to the right direction in FIG. 7), the guide pins 7a on the printing head mounting portion 7 provided with the printing head 9 move along the first guide slot 35 and the second guide slot 37 under the action of the reset spring 41 into the notches located at their ends. At this position, the printing head 9 presses against the rubber platen 27 so that the rubber platen 27 is subjected to a desired pressure and undergoes a desired deformation.

Preferably, the length L of the first guide slot 35 and the second guide slot 37 is selected to be equal to the maximum travel that the movable frame assembly 11 can be pulled out of the housing of the medical monitoring device. In this application, the length of the first guide slot 35 and the second guide slot 37 refers to the shortest linear distance in the horizontal direction between the guide pin 7a on the printing head mounting portion 7 when the movable frame assembly 11 is fully pulled outwardly out of the housing of the medical monitoring device and the guide pin 7a on the printing head mounting portion 7 when the movable frame assembly 11 is fully pushed into the housing of the medical monitoring device. The height H of the first guide slot 35 and the second guide slot 37 is selected to determine the maximum rotation angle of the printing head 9 and the support arm 5. In the present application, the height of the first guide slot 35 and the second guide slot 37 refers to the shortest linear distance in the vertical direction between the guide pin 7a on the printing head mounting portion 7, when the movable frame assembly 11 is fully pulled out of the housing of the medical monitoring device, and the guide pin 7a on the printing head mounting portion 7, when the movable frame assembly 11 is fully pushed into the housing of the medical monitoring device.

In a normal state, the movable frame assembly 11 is pushed into the space between the support plate 3 and the support arm 5 in the housing of the medical monitoring device so that the printing head on the printing head mounting portion 7 is in contact with the rubber platen 27 under pressure. The print paper 16 may pass between the rubber platen 27 and the printing head. When the printing mechanism operates, the electric motor 31 drives the rubber platen 27 to rotate by means of the second pulley, the electric motor 31, and the first pulley 29, thereby dragging thermal print paper past the printing head to record a curve characterizing fetal vital characteristics on the print paper.

When the movable frame assembly 11 is pulled outwardly to pick up and add the print papers, the first guide slot 35 in the first mounting plate 15 and the second guide slot 37 in the second mounting plate 17 of the frame 13 allow the movable frame assembly 11 to move outwardly relative to the support arm 5 and the printing head mounting portion 7, thereby moving the print paper drawer out of the housing of the medical monitoring device to pick up and add the print papers.

According to the present invention, the trajectory of the printing head is determined by the curved contour of the first guide slot and the second guide slot, and the relative position and the compression force between the printing head and the rubber platen is ensured by limiting the relative position of the printing head and the rubber platen. When the moveable frame assembly is pulled outwardly, the moveable frame assembly comprising the rubber platen moves along the first guide slot and the second guide slot, and the printing head slightly rotates along the first guide slot and the second guide slot and is lifted away from the rubber platen as the moveable frame assembly moves. When the movable frame assembly is pushed inwardly, the guide pins on the printing head mounting portion move into the notches at the ends of the first and second guide slots under the action of the reset spring, and the printing head just presses against the rubber platen to achieve the appropriate compression force. The curved contour of the first guide slot and the second guide slot on the one hand ensures that the printing head and the rubber platen accurately cooperate with each other, and the rubber platen is subjected to the appropriate pressure and the appropriate deformation so as to achieve excellent printing quality. On the other hand, the rotation amplitude of the printing head and the support arm is limited, thereby not affecting the layout of the medical monitoring device, and not occupying too much space of the medical monitoring device.

Although the present invention has been described in detail in connection with preferred embodiments, it should be understood that such detailed description is intended only to illustrate the present invention and does not constitute any limit to the present invention which is defined by the appended claims. For example, although in the above preferred embodiments the driving mechanism for driving the rubber platen to rotate comprises the electric motor, the first pulley, the second pulley, and the timing belt, it should be understood that the driving mechanism for driving the rubber platen to rotate may be of other construction, including for example an electric motor mounted on the frame, which drives the rubber platen also mounted on the frame by gear engagement. Thus, the scope of the present invention is defined by the claims.

## Claims

1. A printing mechanism for a medical monitoring device comprising:
a support plate (3);
a L-shaped support arm (5) rotatably disposed on the support plate (3) at one end, the support arm (5) provided with a printing head mounting portion (7) at the other end, the printing head mounting portion (7) provided with a printing head (9) thereon;
**characterized by**
a movable frame assembly (11) movably disposed on the support plate (3) and between the support plate (3) and the support arm (5), the movable frame assembly (11) comprising a frame (13), a rubber platen (27) mounted at a front end of the frame (13) adjacent to the outside of the medical monitoring device to be in contact with the printing head (9) under pressure, a print paper drawer (23) mounted on the frame (13), and a driving mechanism mounted on the frame (13) to drive the rubber platen (27) to rotate;
wherein the frame (13) is formed with a guide slot having a curved contour therein, and a guide pin (7a) extending from both sides of the printing head mounting portion (7) is movably received in the respective guide slot to limit a relative position and a compression force between the printing head (9) and the rubber platen (27).

2. A printing mechanism for a medical monitoring device as claimed in claim 1, wherein the guide slot comprises a first guide slot (35) and a second guide slot (37) formed oppositely in the frame (13), the first guide slot (35) and the second guide slot (37) have a curved contour that rises gradually from a front end to a rear end thereof.

3. A printing mechanism for a medical monitoring device as claimed in claim 2, wherein the first guide slot (35) and the second guide slot (37) have a notch (37a) formed at the front end, and the printing head (9) presses against the rubber platen (27) when the guide pin (7a) is moved into the notch (37a).

4. A printing mechanism for a medical monitoring device as claimed in claim 2, wherein the length (L) of the first guide slot (35) and the second guide slot (37) is selected to be equal to the maximum travel of the movable frame assembly (11) when the movable frame assembly (11) is pulled out of a housing of the medical monitoring device, and the height (H) of the first guide slot (35) and the second guide slot (37) is selected to determine a maximum rotation angle of the printing head (9) and the support arm (5).

5. A printing mechanism for a medical monitoring device as claimed in claim 2, wherein the frame (13) comprises a first mounting plate (15) and a second mounting plate (17) disposed opposite to each other, and a first connecting plate (19) and a second connecting plate (21) connecting fixedly the first mounting plate (15) and the second mounting plate (17) together, the first guide slot (35) is formed in the first mounting plate (15) and the second guide slot (37) is formed in the second mounting plate (17).

6. A printing mechanism for a medical monitoring device as claimed in claim 1, wherein the guide pin (7a) is provided with a rolling bearing thereon, and the guide pin (7a) is movably received in the respective guide slot by the rolling bearing.

7. A printing mechanism for a medical monitoring device as claimed in claim 1, wherein a reset spring (41) is further provided, and the reset spring (41) tends to cause the printing head (9) to rotate and press against the rubber platen (27).

8. A printing mechanism for a medical monitoring device as claimed in claim 5, wherein the driving mechanism comprises a first pulley (29) fixedly mounted at one end of the rubber platen (27), an electric motor (31) mounted on the frame (13), a second pulley mounted on a rotating shaft of the electric motor (31), and a timing belt (33) for connecting the first pulley (29) and the second pulley so as to drive the rubber platen (27) to rotate when the electric motor (31) operates.

9. A printing mechanism for a medical monitoring device as claimed in claim 8, wherein the electric motor (31) is disposed at a rear end of the frame (13) adjacent to the inside of the medical monitoring device.

10. A printing mechanism for a medical monitoring device as claimed in claim 8, wherein the rubber platen (27) is rotatably supported by bearings at a front end of the first mounting plate (15) and the second mounting plate (17) adjacent to the outside of the medical monitoring device, the electric motor (31) is spaced apart from the rubber platen (27) along a longitudinal direction of the first mounting plate (15) and is mounted at a rear end of the first mounting plate (15) adjacent to the inside of the medical monitoring device.

11. A printing mechanism for a medical monitoring device as claimed in claim 8, wherein the first pulley (29) and the second pulley are disposed adjacent to the first mounting plate (15).

12. A printing mechanism for a medical monitoring device as claimed in claim 5, wherein the first mounting plate (15), the second mounting plate (17), the first connecting plate (19), and the second connecting plate (21) are metal or alloy members which are joined together to form a rigid frame .

13. A printing mechanism for a medical monitoring device as claimed in claim 1, wherein the medical monitoring device is a fetal monitor.

14. A medical monitoring device, wherein the medical monitoring device comprises a printing mechanism for a medical monitoring device as claimed in any one of claims 1-13.

## Patentansprüche

1. Druckmechanismus für eine medizinische Überwachungsvorrichtung, umfassend:
eine Tragplatte (3);
einen L-förmigen Tragarm (5), der an einem Ende drehbar auf der Tragplatte (3) angeordnet ist, wobei der Tragarm (5) am anderen Ende mit einem Druckkopf-Montageabschnitt (7) versehen ist, wobei der Druckkopf-Montageabschnitt (7) mit einem darauf befindlichen Druckkopf (9) versehen ist;
**gekennzeichnet durch**:
eine bewegliche Rahmenbaugruppe (11), die beweglich auf der Tragplatte (3) und zwischen der Tragplatte (3) und dem Tragarm (5) angeordnet ist, wobei die bewegliche Rahmenbaugruppe (11) Folgendes umfasst: einen Rahmen (13), eine Gummiwalze (27), die an einem vorderen Ende des Rahmens (13) angrenzend an die Außenseite der medizinischen Überwachungsvorrichtung angebracht ist, um unter Druck mit dem Druckkopf (9) in Kontakt zu sein, eine auf dem Rahmen (13) angebrachte Druckpapier-Schublade (23) und einen auf dem Rahmen (13) angebrachten Antriebsmechanismus, um die Gummiwalze (27) anzutreiben, damit sie sich dreht;
wobei der Rahmen (13) mit einem Führungsschlitz darin ausgebildet ist, der eine gekrümmter Kontur aufweist, und ein Führungsstift (7a), der sich von beiden Seiten des Druckkopf-Montageabschnitts (7) erstreckt, beweglich in dem jeweiligen Führungsschlitz aufgenommen ist, um eine relative Position und eine Kompressionskraft zwischen dem Druckkopf (9) und der Gummiwalze (27) zu begrenzen.

2. Druckmechanismus für eine medizinische Überwachungsvorrichtung nach Anspruch 1, wobei der Führungsschlitz einen ersten Führungsschlitz (35) und einen zweiten Führungsschlitz (37) umfasst, die gegenüberliegend im Rahmen (13) ausgebildet sind, wobei der erste Führungsschlitz (35) und der zweite Führungsschlitz (37) eine gekrümmte Kontur aufweisen, die allmählich von einem vorderen Ende zu einem hinteren Ende derselben ansteigt.

3. Druckmechanismus für eine medizinische Überwachungsvorrichtung nach Anspruch 2, wobei der erste Führungsschlitz (35) und der zweite Führungsschlitz (37) am vorderen Ende ausgebildete Kerbe (37a) aufweisen und der Druckkopf (9) gegen die Gummiwalze (27) drückt, wenn der Führungsstift (7a) in die Kerbe (37a) bewegt wird.

4. Druckmechanismus für eine medizinische Überwachungsvorrichtung nach Anspruch 2, wobei die Länge (L) des ersten Führungsschlitzes (35) und des zweiten Führungsschlitzes (37) so ausgewählt ist, dass sie gleich dem maximalen Betätigungsweg der beweglichen Rahmenbaugruppe (11) ist, wenn die bewegliche Rahmenbaugruppe (11) aus einem Gehäuse der medizinischen Überwachungsvorrichtung herausgezogen wird, und die Höhe (H) des ersten Führungsschlitzes (35) und des zweiten Führungsschlitzes (37) so ausgewählt ist, dass sie einen maximalen Drehwinkel des Druckkopfes (9) und des Tragarms (5) bestimmt.

5. Druckmechanismus für eine medizinische Überwachungsvorrichtung nach Anspruch 2, wobei der Rahmen (13) eine erste Montageplatte (15) und eine zweite Montageplatte (17), die einander gegenüberliegend angeordnet sind, und eine erste Verbindungsplatte (19) und eine zweite Verbindungsplatte (21), welche die erste Montageplatte (15) und die zweite Montageplatte (17) fest miteinander verbinden, umfasst, wobei der erste Führungsschlitz (35) in der ersten Montageplatte (15) ausgebildet ist und der zweite Führungsschlitz (37) in der zweiten Montageplatte (17) ausgebildet ist.

6. Druckmechanismus für eine medizinische Überwachungsvorrichtung nach Anspruch 1, wobei der Führungsstift (7a) mit einem Wälzlager darauf versehen ist und der Führungsstift (7a) durch das Wälzlager beweglich in dem jeweiligen Führungsschlitz aufgenommen ist.

7. Druckmechanismus für eine medizinische Überwachungsvorrichtung nach Anspruch 1, wobei außerdem eine Rückholfeder (41) bereitgestellt ist und die Rückholfeder (41) danach strebt, den Druckkopf (9) zu drehen und gegen die Gummiwalze (27) zu drücken.

8. Druckmechanismus für eine medizinische Überwachungsvorrichtung nach Anspruch 5, wobei der Antriebsmechanismus Folgendes umfasst: eine erste Riemenscheibe (29), die fest an einem Ende der Gummiwalze (27) angebracht ist, einen Elektromotor (31), der an dem Rahmen (13) angebracht ist, eine zweite Riemenscheibe, die auf einer Drehwelle des Elektromotors (31) angebracht ist, und einen Zahnriemen (33) zum Verbinden der ersten Riemenscheibe (29) und der zweiten Riemenscheibe, um die Gummiwalze (27) anzutreiben, damit sie sich dreht, wenn der Elektromotor (31) läuft.

9. Druckmechanismus für eine medizinische Überwachungsvorrichtung nach Anspruch 8, wobei der Elektromotor (31) an einem hinteren Ende des Rahmens (13) angrenzend an die Innenseite der medizinischen Überwachungsvorrichtung angeordnet ist.

10. Druckmechanismus für eine medizinische Überwachungsvorrichtung nach Anspruch 8, wobei die Gummiwalze (27) durch Lager an einem vorderen Ende der ersten Montageplatte (15) und der zweiten Montageplatte (17) angrenzend an die Außenseite der medizinischen Überwachungsvorrichtung drehbar gelagert ist, der Elektromotor (31) in einer Längsrichtung der ersten Montageplatte (15) von der Gummiwalze (27) beabstandet ist und an einem hinteren Ende der ersten Montageplatte (15) angrenzend an die Innenseite der medizinischen Überwachungsvorrichtung angebracht ist.

11. Druckmechanismus für eine medizinische Überwachungsvorrichtung nach Anspruch 8, wobei die erste Riemenscheibe (29) und die zweite Riemenscheibe angrenzend an die erste Montageplatte (15) angeordnet sind.

12. Druckmechanismus für eine medizinische Überwachungsvorrichtung nach Anspruch 5, wobei die erste Montageplatte (15), die zweite Montageplatte (17), die erste Verbindungsplatte (19) und die zweite Verbindungsplatte (21) Metall- oder Legierungselemente sind, die miteinander verbunden sind, um einen starren Rahmen zu bilden.

13. Druckmechanismus für eine medizinische Überwachungsvorrichtung nach Anspruch 1, wobei die medizinische Überwachungsvorrichtung ein Fetalmonitor ist.

14. Medizinische Überwachungsvorrichtung, wobei die medizinische Überwachungsvorrichtung einen Druckmechanismus für eine medizinische Überwachungsvorrichtung nach einem der Ansprüche 1 bis 13 umfasst.

## Revendications

1. Mécanisme d'impression destiné à un dispositif de surveillance médicale comprenant :
une plaque de support (3) ;
un bras de support en forme de L (5) disposé en rotation sur la plaque de support (3) à une extrémité, le bras de support (5) étant pourvu d'une partie de montage de tête d'impression (7) à l'autre extrémité, la partie de montage de tête d'impression (7) étant pourvue d'une tête d'impression (9) sur celle-ci ;
**caractérisé par**
un ensemble cadre mobile (11) disposé de manière mobile sur la plaque de support (3) et entre la plaque de support (3) et le bras de support (5), l'ensemble cadre mobile (11) comprenant un cadre (13), une plaque en caoutchouc (27) montée à une extrémité avant du cadre (13) adjacente à l'extérieur du dispositif de surveillance médicale pour être en contact avec la tête d'impression (9) sous pression, un tiroir à papier d'impression (23) monté sur le cadre (13), et un mécanisme d'entraînement monté sur le cadre (13) pour entraîner la plaque en caoutchouc (27) en rotation ;
dans lequel le cadre (13) est formé avec une fente de guidage présentant un contour incurvé à l'intérieur, et une broche de guidage (7a) s'étendant des deux côtés de la partie de montage de tête d'impression (7) est reçue de manière mobile dans la fente de guidage respective pour limiter une position relative et une force de compression entre la tête d'impression (9) et le plateau en caoutchouc (27).

2. Mécanisme d'impression pour un dispositif de surveillance médicale selon la revendication 1, dans lequel la fente de guidage comprend une première fente de guidage (35) et une seconde fente de guidage (37) formées de manière opposée dans le cadre (13), la première fente de guidage (35) et la seconde fente de guidage (37) présentent un contour incurvé qui s'élève progressivement depuis une extrémité avant jusqu'à une extrémité arrière de celle-ci.

3. Mécanisme d'impression pour un dispositif de surveillance médicale selon la revendication 2, dans lequel la première fente de guidage (35) et la seconde fente de guidage (37) présentent une encoche (37a) formée à l'extrémité avant et la tête d'impression (9) appuie contre la platine en caoutchouc (27) lorsque la broche de guidage (7a) est déplacée dans l'encoche (37a).

4. Mécanisme d'impression pour un dispositif de surveillance médicale selon la revendication 2, dans lequel la longueur (L) de la première fente de guidage (35) et de la seconde fente de guidage (37) est sélectionnée pour être égale à la course maximale de l'ensemble cadre mobile (11) lorsque l'ensemble cadre mobile (11) est retiré d'un boîtier du dispositif de surveillance médicale, et la hauteur (H) de la première fente de guidage (35) et de la seconde fente de guidage (37) est sélectionnée pour déterminer un angle de rotation maximal de la tête d'impression (9) et du bras de support (5).

5. Mécanisme d'impression pour un dispositif de surveillance médicale selon la revendication 2, dans lequel le cadre (13) comprend une première plaque de montage (15) et une seconde plaque de montage (17) disposées l'une en face de l'autre, et une première plaque de liaison (19) et une seconde plaque de liaison (21) reliant à demeure la première plaque de montage (15) et la seconde plaque de montage (17) ensemble, la première fente de guidage (35) est formée dans la première plaque de montage (15) et la seconde fente de guidage (37) est formée dans la seconde plaque de montage (17).

6. Mécanisme d'impression pour un dispositif de surveillance médicale selon la revendication 1, dans lequel la broche de guidage (7a) est pourvue d'un palier à roulement, et la broche de guidage (7a) est reçue de manière mobile dans la fente de guidage respective par le palier à roulement.

7. Mécanisme d'impression pour un dispositif de surveillance médicale selon la revendication 1, dans lequel un ressort de réinitialisation (41) est en outre prévu et le ressort de réinitialisation (41) tend à amener la tête d'impression (9) à tourner et à appuyer contre le plateau en caoutchouc (27).

8. Mécanisme d'impression pour un dispositif de surveillance médicale selon la revendication 5, dans lequel le mécanisme d'entraînement comprend une première poulie (29) montée à demeure à une extrémité du plateau en caoutchouc (27), un moteur électrique (31) monté sur le cadre (13), une seconde poulie montée sur un arbre rotatif du moteur électrique (31), et une courroie de distribution (33) pour relier la première poulie (29) et la seconde poulie de manière à entraîner le plateau en caoutchouc (27) en rotation lorsque le moteur électrique (31) fonctionne.

9. Mécanisme d'impression pour un dispositif de surveillance médicale selon la revendication 8, dans lequel le moteur électrique (31) est disposé à une extrémité arrière du cadre (13) adjacente à l'intérieur du dispositif de surveillance médicale.

10. Mécanisme d'impression pour un dispositif de surveillance médicale selon la revendication 8, dans lequel le plateau en caoutchouc (27) est supporté en rotation par des paliers à une extrémité avant de la première plaque de montage (15) et de la seconde plaque de montage (17) adjacente à l'extérieur du dispositif de surveillance médicale, le moteur électrique (31) est espacé du plateau en caoutchouc (27) le long d'une direction longitudinale de la première plaque de montage (15) et est monté à une extrémité arrière de la première plaque de montage (15) adjacente à l'intérieur du dispositif de surveillance médicale.

11. Mécanisme d'impression pour un dispositif de surveillance médicale selon la revendication 8, dans lequel la première poulie (29) et la seconde poulie sont disposées adjacentes à la première plaque de montage (15).

12. Mécanisme d'impression pour un dispositif de surveillance médicale selon la revendication 5, dans lequel la première plaque de montage (15), la seconde plaque de montage (17), la première plaque de liaison (19) et la seconde plaque de liaison (21) sont des éléments en métal ou en alliage qui sont joints ensemble pour former un cadre rigide.

13. Mécanisme d'impression pour un dispositif de surveillance médicale selon la revendication 1, dans lequel le dispositif de surveillance médicale est un moniteur fœtal.

14. Dispositif de surveillance médicale, dans lequel le dispositif de surveillance médicale comprend un mécanisme d'impression pour un dispositif de surveillance médicale selon l'une quelconque des revendications 1-13.
